# DEMANDE DE BREVET EUROPEEN

(11) **EP 1 491 181 A2**
(43) Date de publication de la demande: **29.12.2004**
(21) Numéro de dépôt: 04300403.5
(22) Date de dépôt: 28.06.2004
(51) Int. Cl.: A61K 7/09

(54) **Composition cosmétique à base de précurseur(s) de radical thiyl pour la déformation permanente des fibres kératiniques**

(30) Priorité: 27.06.2003 FR 0350267
(71) Demandeur: L'OREAL, 75008 Paris (FR)
(72) Inventeur: Vic, Gabin, 60280, VENETTE (FR); Livoreil, Aude, 75006, PARIS (FR); Samain, Henri, 91570, BIEVRES (FR)
(74) Mandataire: Michelet, Alain

(57) **Abrégé**

L'invention concerne une composition cosmétique pour la déformation permanente des fibres kératiniques, en particulier des cheveux, comprenant, dans un milieu cosmétiquement acceptable, au moins un précurseur de radical thiyl.

## Description

L'invention se rapporte à une composition cosmétique pour la déformation permanente des fibres kératiniques, en particulier des cheveux.

Généralement, la technique utilisée pour obtenir une déformation permanente des cheveux consiste, dans un premier temps, à réaliser l'ouverture des liaisons disulfures -S-S- de la kératine (cystine) en appliquant sur les cheveux préalablement mis sous tension (bigoudis et autres) une composition réductrice (étape de réduction) puis, de préférence après avoir rincé la chevelure ainsi traitée, à reconstituer dans un second temps lesdites liaisons disulfures en appliquant sur les cheveux toujours sous tension une composition oxydante (étape d'oxydation, dite aussi de fixation) de façon à donner finalement aux cheveux la forme recherchée. Cette technique permet ainsi de réaliser indifféremment soit l'ondulation des cheveux, soit leur défrisage ou leur décrêpage. La nouvelle forme imposée aux cheveux par un traitement chimique tel que ci-dessus est durable dans le temps et résiste notamment à l'action des lavages à l'eau ou par shampooings, et ceci par opposition aux simples techniques classiques de déformation temporaires, telles que les techniques de mise en pli.

Les compositions réductrices utilisables pour la mise en oeuvre de la première étape d'un tel procédé classique de déformation permanente des cheveux (procédé en deux temps) contiennent généralement des composés thiolés, tels que l'acide thioglycolique ou la cystéine, ou des composés générant des ions hydroxydes, tels que la soude ou le carbonate de guanidine par exemple.

Les compositions oxydantes utilisables pour l'étape de fixation sont généralement à base d'eau oxygénée ou de bromates alcalins.

Cependant, une telle technique ne donne pas entièrement satisfaction. En effet, cette technique est très efficace pour modifier la forme des cheveux, mais très dégradante pour les fibres capillaires.

Ainsi, lorsqu'une permanente à l'acide thioglycolique est appliquée sur des cheveux ayant subi une coloration, un très fort décapage de la couleur est observé et la surface des cheveux est fortement dégradée. A l'inverse, si une coloration est appliquée sur des cheveux permanentés, la couleur obtenue est très différente de la couleur normalement obtenue sur des cheveux naturels non permanentés.

Par ailleurs, dans le cas d'un défrisage à la soude, on observe généralement des phénomènes importants de cassure des cheveux.

La présente invention vise donc à résoudre les problèmes ci-dessus, et à fournir une composition cosmétique pour la déformation permanente des cheveux qui réduise fortement la dégradation des cheveux et soit pratiquement sans effet sur une coloration artificielle des fibres kératiniques.

La Demanderesse a trouvé de façon surprenante qu'il était possible de formuler une composition cosmétique remédiant aux inconvénients de l'art antérieur, en utilisant dans un milieu cosmétiquement acceptable au moins un précurseur de radical thiyl.

La présente invention concerne donc une composition cosmétique pour la déformation permanente des fibres kératiniques, en particulier des cheveux, comprenant, dans un milieu cosmétiquement acceptable, au moins un précurseur de radical thiyl.

Par déformation permanente, on entend le frisage permanent (encore appelé permanente), le défrisage ou le décrêpage des fibres kératiniques.

Par précurseur de radical thiyl, on entend un composé capable, dans les conditions d'utilisation, de fournir un radical RS° (radical thiyl), où R est tel que défini ci-dessous.

Selon l'invention, le ou les précurseurs de radical thiyl sont de formule :

R-(S-X)ₙ

où
n est un entier variant de 1 à 5,
R est un groupe hydrocarboné, linéaire, ramifié ou cyclique, saturé ou insaturé, pouvant être interrompu par un ou plusieurs hétéroatomes choisis parmi le soufre, l'oxygène, l'azote, le silicium ou le phosphore, et pouvant comprendre un ou plusieurs substituants choisis parmi les groupes hydroxyle, amine, carbamate, carbonate, hydrazine, éther, acide carboxylique, ester, amide, cyano et uréido, et
X est :
- soit un halogène, tel que le fluor, le brome, le chlore ou l'iode,
- soit un groupe -NO, étant entendu que R-(S-X)ₙ n'est pas la S-nitroso-thioglycérine,
- soit un groupe -C(O)R", -C(S)R" ou -C(N)R", où R" est un groupe hydrocarboné, linéaire, ramifié ou cyclique, saturé ou insaturé, pouvant être interrompu par un ou plusieurs hétéroatomes choisis parmi le soufre, l'oxygène, l'azote, le silicium ou le phosphore, et pouvant comprendre un ou plusieurs substituants choisis parmi les groupes hydroxyle, amine, carbamate, carbonate, hydrazine, éther, acide carboxylique, ester, amide, cyano et uréido, R" pouvant être identique ou différent de R.

Lorsque X est un groupe -NO, le ou les précurseurs de radical thiyl sont alors des nitrosothiols et sont choisis de préférence parmi les S-nitroso-aminoacides, les oligosaccharides S-nitrosylés, les polysaccharides S-nitrosylés, les S-nitroso-polypeptides et les S-nitroso-oligonucléotides.

Parmi les S-nitroso-aminoacides utilisables dans la composition selon l'invention, on peut citer les composés de formule :

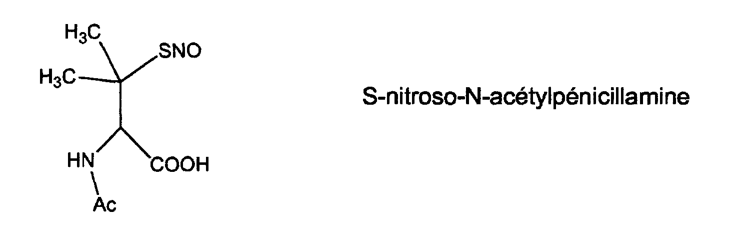

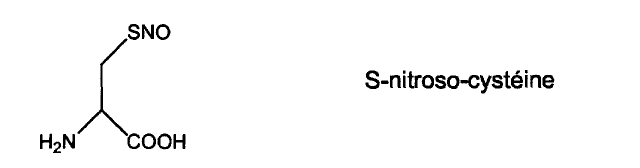

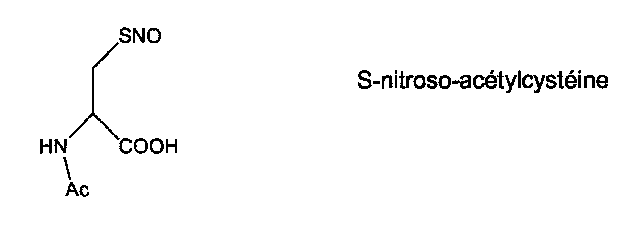

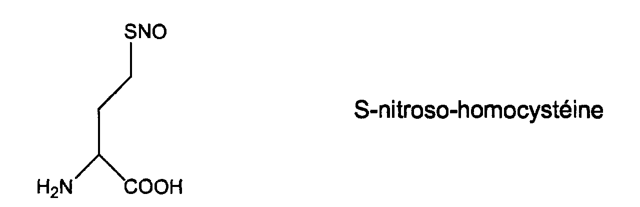

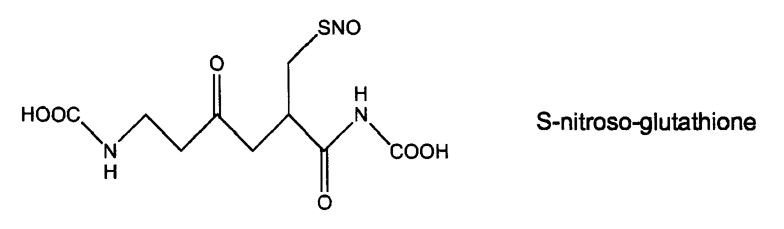

Lorsque le ou les nitrosothiols sont des S-nitroso-polypeptides, ils peuvent être choisis parmi les protéines S-nitrosylées.

Lorsque le ou les nitrosothiols sont des S-nitroso-oligonucléotides, ils peuvent être choisis parmi l'acide désoxyribonucléique S-nitrosylé et l'acide ribonucléique S-nitrosylé.

Le ou les nitrosothiols utilisables dans la composition selon l'invention peuvent encore être choisis parmi les composés de formule :

HOOC-CH₂-CH₂-SNO

HOOC-CH₂-SNO

H₂N-CH₂-CH₂-SNO

HO-CH₂-CH₂-SNO

ONS-CH₂-CH(OH)-CH(OH)-CH₂-SNO

Ac-NH-CH₂-C(CH₃)₂-SNO

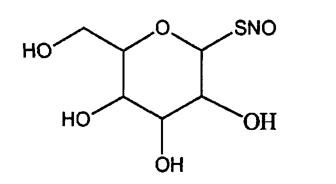

Le ou les précurseurs de radical thiyl utilisés dans la composition selon l'invention sont généralement préparés suivant les modes opératoires décrits dans les références suivantes : Can. J. Chem. 1999, vol 77, p. 550-556, et J. Chem. Soc., Chem. Commun. 1978, p.249.

Avantageusement, le ou les précurseurs de radical thiyl représentent de 0,001 à 30 %, de préférence de 0,01 à 15 %, mieux de 0,1 à 10 %, en poids par rapport au poids total de la composition.

Le radical thiyl peut être généré à partir des précurseurs de radical thiyl soit spontanément, soit sous l'action d'un agent chimique, tel que des sels métalliques ou des agents modifiant le pH, soit sous l'action d'un agent physique, tel que la chaleur ou les rayonnements UV, IR ou micro-ondes.

En outre, la composition selon l'invention comprend avantageusement au moins un sel de nitrosonium.

En général, le ou les sels de nitrosonium utilisables dans les compositions selon l'invention sont de formule :

m NO⁺, Y^{m-}

où m est un entier variant de 1 à 5, et
où Y^{m-} est un anion minéral ou organique, de préférence choisi parmi les anions halogénure, tels que fluorure, bromure, iodure, chlorure, les anions nitrate, nitrite, carbonate, phosphate, phosphonate, sulfate, sulfite, acétate, tétrafluoroborate, perchlorate, ClO₃⁻, BrO₃⁻, FeCl₄⁻, FeCl₂⁻, CF₃CO₂⁻, AlCl₄⁻, KS₂O₈⁻, HSO₄⁻, CF₃SO₃⁻, CH₃SO₃⁻, CH₃C₆H₄SO₃⁻, hexachloroantimonate et hexafluorophosphate.

De préférence, le ou les sels de nitrosonium représentent de 0,001 à 30 %, en poids par rapport au poids total de la composition.

Selon un mode de réalisation préféré, la composition selon l'invention comprend en outre au moins un thiol et/ou au moins un sulfite et/ou au moins bisulfite.

Le ou les thiols utilisables dans la composition selon l'invention peuvent être des monothiols ou des dithiols.

Les monothiols sont de préférence choisis parmi l'acide thioglycolique, les esters de l'acide thioglycolique, l'acide thiolactique, la cystéine, les dérivés de la cystéine tel que la N-acétylcystéine, la cystéamine et les dérivés de la cystéamine, tel que la N-acétylcystéamine.

A titre de dithiol, on peut citer la bis mercapto éthylsulfone.

De préférence, les thiols représentent de 0,001 à 30 %, en poids par rapport au poids total de la composition.

A titre de sulfite utilisable dans la composition selon l'invention, on peut citer le sulfite de sodium. A titre de bisulfite, on peut citer le bisulfite de sodium.

De préférence, le ou les sulfites ou bisulfites représentent de 0,001 à 30 % en poids par rapport au poids total de la composition.

La composition selon l'invention peut également comprendre au moins un chélateur de métaux et/ou au moins un agent réducteur autre que les thiols, dithiols, sulfites et bisulfites décrits ci-dessus.

Par chélateurs de métaux, on entend les composés chimiques ou biologiques ayant la capacité de perturber et de modifier l'environnement ionique en séquestrant les ions métalliques.

Comme exemples de chélateurs de métaux chimiques utilisables dans la composition selon l'invention, on peut citer :
- l'acide aminotriméthyle phosphonique,
- l'acide β-alanine diacétique,
- l'acide citrique,
- la cyclodextrine,
- l'acide cyclohexanediamine tétracétique,
- l'acide diéthylènetriamine pentaméthylène phosphonique,
- l'acide diéthanolamine N-acétique (diéthanolamine N-acétique),
- l'acide éthylène diamine tétracétique (EDTA ou YH₄) et ses sels de sodium (YH₃Na, Y₂H₂Na₂, YH₃Na₃ et YNa₄), de potassium (YH₃K, Y₂H₃K₃ et YK₄), de calcium, de disodium, de diammonium et ses sels de triéthanolamine (TEA-EDTA),
- l'acide étidronique,
- l'acide galactanique,
- l'acide hydroxyéthyl éthylènediamine tétracétique (HEDTA) et son sel trisodique,
- l'acide gluconique,
- l'acide glucuronique,
- l'acide nitrilotriacétique (NTA) et son sel trisodique,
- l'acide pentétique,
- l'acide phytique,
- l'acide ribonique,
- le citrate de diammonium,
- l'azacycloheptane diphosphonate de disodium,
- le pyrophoshate de disodium,
- l'hydroxypropyl cyclodextrine,
- le méthyl cyclodextrine,
- le triphosphate de pentapotassium,
- l'aminotriméthylène phosphonate de pentasodium,
- l'éthylènediamine tétraméthylène phosphonate de pentasodium,
- le pentétate de pentasodium,
- le triphosphate de pentasodium,
- le citrate de potassium,
- EDTMP de potassium,
- EDTMP de sodium,
- le chitosane méthylène phosphonate de sodium,
- l'hexamétaphosphate de sodium,
- le métaphosphate de sodium,
- le polyphosphate de potassium,
- le polyphosphate de sodium,
- le trimétaphosphate de sodium,
- le dihydroxyéthylglycinate de sodium,
- le gluconate de potassium,
- le gluconate de sodium,
- le glucopeptate de sodium,
- le glycereth-1 polyphosphate de sodium,
- le pyrophosphate de tétrapotassium,
- le polyphosphate de triéthanolamine (TEA),
- le pyrophosphate de tétrasodium,
- le phosphate de trisodium,
- l'oxyde triphosphonométhylamine de potassium,
- le métasilicate de sodium,
- le phytate de sodium,
- le polydiméthylglycinophénolsulfonate de sodium,
- le tétrahydroxyéthyl éthylène diamine,
- le tétrahydroxypropyl éthylène diamine,
- l'étidronate de tétrapotassium,
- l'étidronate de tétrasodium,
- l'iminodisuccinate de tétrasodium,
- l'éthylènediamine disuccinate de trisodium,
- l'acide éthanolamine N,N-diacétique,
- l'acétate de disodium,
- le dimercaprol,
- la déferoxamine,
- le Zylox, chélateur du fer décrit et revendiqué dans la demande internationale WO 94/61338,
- la néocuproine.

Comme autres exemples de chélateurs de métaux utilisables dans la composition selon l'invention, on peut citer la métallothionéine, la transferrine, la calmoduline, et le chitosane méthylène phosphonate de sodium.

De préférence, le ou les chélateurs de métaux sont choisis parmi l'acide éthylène diamine tétracétique (EDTA) et ses sels de sodium, de potassium, de calcium, de disodium, de diammonium et de triéthanolamine (TEA-EDTA), l'acide hydroxyéthyl éthylène diamine tétracétique (HEDTA) et son sel trisodique, et la néocuproine.

En général, le ou les chélateurs de métaux représentent de 0,00001 à 10 %, en poids par rapport au poids total de la composition.

Comme expliqué précédemment, la composition selon l'invention peut comprendre, outre le ou les chélateurs de métaux, un ou plusieurs agents réducteurs.

Le ou les agents réducteurs utilisables dans la composition selon l'invention, autres que les thiols, dithiols, sulfites et bisulfites décrits précédemment, sont de préférence choisis parmi les (C₁-C₈) alkyl-phosphines, les réductones dont l'acide ascorbique et l'acide érythorbique.

En général, le ou les agents réducteurs ci-dessus représentent de 0,001 à 30 %, en poids par rapport au poids total de la composition.

Le ou les constituants de la composition selon l'invention doivent être placés dans un milieu cosmétiquement acceptable.

Pour cela, on choisit généralement un ou plusieurs solvants classiquement utilisés dans les compositions cosmétiques. On peut citer les alcools en C₁-C₆, de préférence les alcanols tels que l'éthanol, le propanol et l'isopropanol, les alcanediols tels que l'éthyléneglycol, le propylène glycol et le pentanediol, l'alcool benzylique, les éthers en C₁-C₆, les esters en C₁-C₆, la N-méthylpyrrolidone (NMP) et les cétones en C₁-C₆.

De plus, avantageusement, le pH de la solution sera compris entre 2 et 13.

Le réglage du pH de la composition peut être obtenu à l'aide d'un agent alcalin, tel que, par exemple, l'ammoniaque, la monoéthanolamine, la diéthanolamine, la triéthanolamine, la 1,3-propanediamine, un carbonate ou bicarbonate alcalin ou d'ammonium, un carbonate organique tel que le carbonate de guanidine, un hydroxyde alcalin, ou bien à l'aide d'un agent acidifiant tel que par exemple l'acide chlorhydrique, l'acide acétique, l'acide lactique, l'acide oxalique ou l'acide borique.

La composition selon l'invention comprend de préférence au moins un adjuvant cosmétiquement acceptable classiquement utilisé dans les compositions cosmétiques destinées à être appliquées sur les fibres kératiniques. On peut citer en particulier les gélifiants et/ou épaississants, les tensioactifs anioniques, non ioniques, cationiques ou amphotères, les agents propénétrants, les émulsionnants, les parfums, les conservateurs, les charges, les filtres solaires, les matières colorantes, les protéines, les vitamines, les provitamines, les polymères non fixants anioniques, non ioniques, cationiques ou amphotères, les agents hydratants, les émollients, les agents adoucissants, les huiles minérales, végétales ou synthétiques, les actifs hydrophiles ou lipophiles comme les céramides et les pseudocéramides, les agents anti-mousse, les agents antiperspirants, les agents anti-radicaux libres, les polymères fixants ou non, les agents bactéricides et les agents anti-pelliculaires.

La composition selon l'invention peut se présenter sous la forme d'une lotion, épaissie ou non, d'une crème, épaissie ou non, d'un gel ou de toute autre forme appropriée. Elle peut éventuellement être conditionnée dans un flacon pompe ou dans un récipient aérosol.

Lorsque la composition selon l'invention est destinée à une opération de défrisage ou de décrêpage des cheveux, la composition se présente de préférence sous forme d'une crème épaissie, par exemple une crème comprenant au moins un composé choisi parmi le stéarate de glycéryle, le stéarate de glycol, une cire auto-émulsionnable ou un alcool gras, ou bien sous la forme d'un liquide ou d'un gel comprenant un ou plusieurs agents épaississants tels que des polymères ou des copolymères carboxyvinyliques qui collent les cheveux et les maintiennent dans la position lisse pendant le temps de pose.

L'invention concerne encore un procédé de déformation permanente des fibres kératiniques, en particulier des cheveux.

Bien que l'exposé qui suit s'articule essentiellement autour du cas particulier du traitement du cheveu, on notera ici que le procédé selon l'invention est applicable à toute fibre kératinique en général, notamment cils, moustaches, poils et autres.

Dans une première étape, une composition selon l'invention est appliquée sur les fibres kératiniques.

Les cheveux sont mis en forme en utilisant des moyens mécaniques bien connus de l'homme du métier.

Lorsque l'on souhaite réaliser une permanente, on utilise par exemple des bigoudis, la composition selon l'invention étant appliquée avant ou après la pose des bigoudis. De préférence, la composition est appliquée sur des cheveux mouillés préalablement enroulés sur des rouleaux ayant de 4 à 20 mm de diamètre. La composition peut également être appliquée au fur et à mesure de l'enroulage des cheveux.

Lorsque l'on souhaite effectuer le défrisage ou le décrêpage des cheveux, on soumet les cheveux, après application de la composition selon l'invention, à une déformation mécanique permettant de les fixer dans leur nouvelle forme, par une opération de lissage des cheveux avec un peigne à larges dents, avec le dos d'un peigne ou à la main.

Après l'application de la composition selon l'invention, on laisse poser ladite composition et ladite solution, de préférence pendant 5 à 60 minutes, mieux pendant 5 à 30 minutes, soit à l'air libre, soit à la chaleur, à une température comprise entre 20 et 100°C.

Les cheveux sont alors rincés soigneusement.

La dernière étape du procédé selon l'invention consiste à réaliser la fixation des fibres kératiniques, par oxydation. Cette étape peut se faire soit par mise en contact desdites fibres avec l'oxygène de l'air, soit par application d'une composition comprenant au moins un agent d'oxydation, en général une solution diluée de peroxyde d'hydrogène.

Dans un mode de réalisation préféré du procédé selon l'invention, on applique, après l'application de la composition selon l'invention et avant le temps de pose, une composition comprenant au moins un sel et/ou oxyde métallique, par exemple un sel et/ou oxyde de Cu(I), Cu(II) ou Fe(II).

Comme sels et/ou oxydes de Cu(I), Cu(II) ou Fe(II) utilisables selon l'invention, on peut citer par exemple Cu₂NO₂, Cu₂O, CuSO₄, CuBr, FeO, FeCl₂ et Fe(C₃H₅O₃)₂.

Dans ce cas, avantageusement, après avoir laissé poser la composition selon l'invention et la solution comprenant au moins un sel et/ou oxyde métallique, on ajoute une solution comprenant au moins un chélateur de métaux. On laisse alors généralement poser, à température ambiante ou sous la chaleur à une température comprise entre 20 et 100°C.

Les étapes de rinçage et de fixation sont alors les mêmes que précédemment.

Le procédé selon l'invention peut être avantageusement mis en oeuvre en utilisant un kit cosmétique, également objet de la présente invention, comprenant une première composition cosmétique telle que définie précédemment et une seconde composition cosmétique comprenant au moins un sel et/ou oxyde métallique, de préférence un sel et/ou oxyde de Cu(I), Cu(II) ou Fe(II), ladite première composition et ladite deuxième composition étant conditionnées séparément dans des compartiments imperméables à l'oxygène.

Selon un mode de réalisation préféré, ledit kit peut en outre comprendre une troisième composition comprenant au moins un chélateur de métaux, ladite troisième composition étant conditionnée séparément dans des compartiments imperméables à l'oxygène.

La présente invention est illustrée par les exemples suivants.

### Exemples

Des mèches de cheveux natures sont mis en forme en utilisant des bigoudis, puis traités avec une composition cosmétique selon invention dont la formulation est la suivante :
- S-nitroso-N-acétylpénicillamine : 0,5 g
- Eau : qsp 100 g

On laisse poser pendant une heure à température ambiante, on rince les cheveux à l'eau, puis on les sèche au casque.

On obtient une mise en forme des cheveux qui résiste aux shampooings.

## Revendications

1. Composition cosmétique pour la déformation permanente des fibres kératiniques, en particulier des cheveux, **caractérisée en ce qu'**elle comprend, dans un milieu cosmétiquement acceptable, au moins un précurseur de radical thiyl de formule :
R-(S-X)ₙ
où
n est un entier variant de 1 à 5,
R est un groupe hydrocarboné, linéaire, ramifié ou cyclique, saturé ou insaturé, pouvant être interrompu par un ou plusieurs hétéroatomes choisis parmi le soufre, l'oxygène, l'azote, le silicium ou le phosphore, et pouvant comprendre un ou plusieurs substituants choisis parmi les groupes hydroxyle, amine, carbamate, carbonate, hydrazine, éther, acide carboxylique, ester, amide, cyano et uréido, et
X est :
- soit un halogène, tel que le fluor, le brome, le chlore ou l'iode,
- soit un groupe -NO, étant entendu que R-(S-X)ₙ n'est pas la S-nitroso-thioglycérine,
- soit un groupe -C(O)R", -C(S)R" ou -C(N)R", où R" est un groupe hydrocarboné, linéaire, ramifié ou cyclique, saturé ou insaturé, pouvant être interrompu par un ou plusieurs hétéroatomes choisis parmi le soufre, l'oxygène, l'azote, le silicium ou le phosphore, et pouvant comprendre un ou plusieurs substituants choisis parmi les groupes hydroxyle, amine, carbamate, carbonate, hydrazine, éther, acide carboxylique, ester, amide, cyano et uréido, R" pouvant être identique ou différent de R.

2. Composition selon la revendication 1 **caractérisée en ce que**, lorsque X est un groupe -NO, le ou les précurseurs de radical thiyl sont choisis parmi les S-nitroso-aminoacides, les otigosacchandes S-nitrosylés, les polysaccharides S-nitrosylés, les S-nitroso-polypeptides et les S-nitroso-oligonucléotides.

3. Composition selon la revendication 2 **caractérisée en ce que** les S-nitroso-aminoacides sont choisis parmi les composés de formule :

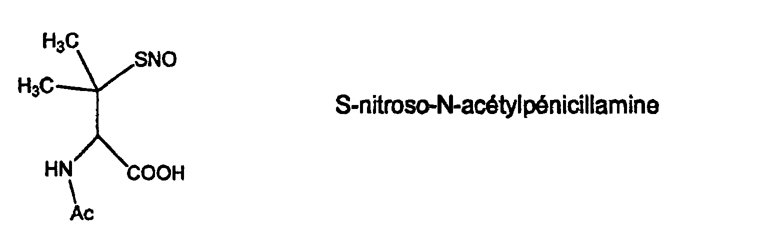

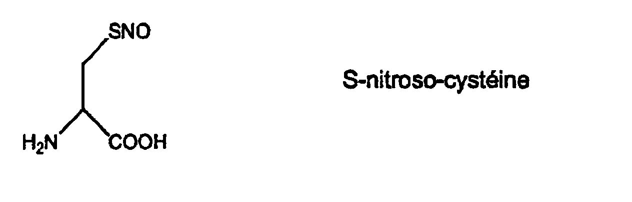

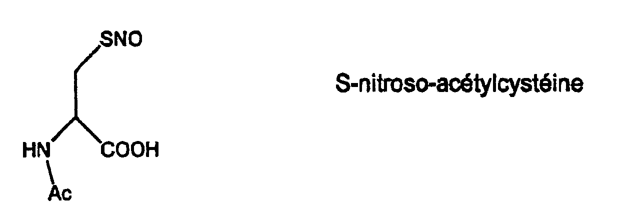

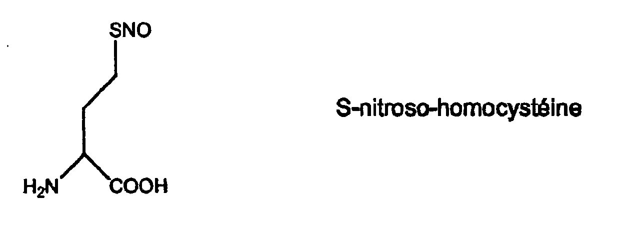

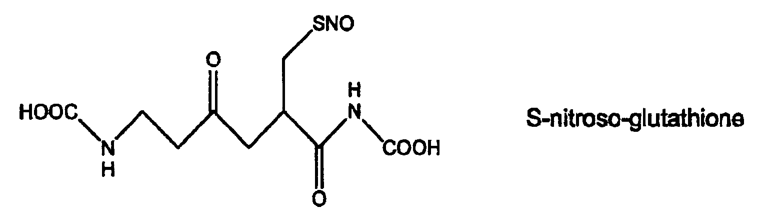

4. Composition selon la revendication 2 **caractérisée en ce que** les S-nitroso-polypeptides sont choisis parmi les protéines S-nitrosylées.

5. Composition selon la revendication 2 **caractérisée en ce que** les S-nitroso-oligonucléotides sont choisis parmi l'acide désoxyribonucléique S-nitrosylé et l'acide ribonucléique S-nitrosylé.

6. Composition selon la revendication 1 **caractérisée en ce que**, lorsque X est un groupe -NO, le ou les précurseurs de radical thiyl sont choisis parmi les composés de formule :
HOOC-CH₂-CH₂-SNO
HOOC-CH₂-SNO
H₂N-CH₂-CH₂-SNO
HO-CH₂-CH₂-SNO
ONS-CH₂-CH(OH)-CH(OH)-CH₂-SNO
Ac-NH-CH₂-C(CH₃)₂-SNO

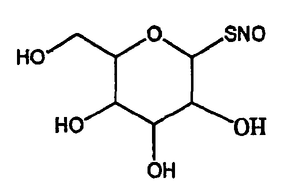

7. Composition selon l'une quelconque des revendications 1 à 6 **caractérisée en ce que** le ou les précurseurs de radical thiyl représentent de 0,001 à 30 %, de préférence de 0,01 à 15 %, mieux de 0,1 à 10 % en poids par rapport au poids total de la composition.

8. Composition selon l'une quelconque des revendications précédentes **caractérisée en ce qu'**elle comprend au moins un sel de nitrosonium.

9. Composition selon la revendication 8 **caractérisée en ce que** le ou les sels de nitrosonium sont de formule :
m NO⁺, Y^{m-}
où m est un entier variant de 1 à 5,
où Y^{m-} est un anion minéral ou organique, de préférence choisi parmi les anions halogénure, tels que fluorure, bromure, iodure, chlorure, les anions nitrate, nitrite, carbonate, phosphate, phosphonate, sulfate, sulfite, acétate, tétrafiuoroborate, perchlorate, ClO₃⁻, BrO₃⁻, FeCl₄⁻, FeCl₂⁻, CF₃CO₂⁻, AlCl₄⁻, KS₂O₈⁻, HSO₄⁻, CF₃SO₃⁻, CH₃SO₃⁻, CH₃C₆H₄SO₃⁻, hexachloroantimonate et hexafluorophosphate.

10. Composition selon la revendication 8 ou 9 **caractérisée en ce que** le ou les sels de nitrosonium représentent de 0,001 à 30 % en poids par rapport au poids total de la composition.

11. Composition selon l'une quelconque des revendications précédentes **caractérisée en ce qu'**elle comprend au moins un thiol et/ou au moins un sulfite et/ou au moins un bisulfite.

12. Composition selon la revendication 11 **caractérisée en ce que** le ou les thiols sont des monothiols ou des dithiols.

13. Composition selon la revendication 12 **caractérisée en ce que** le ou les monothiols sont choisis parmi l'acide thioglycolique, les esters de l'acide thioglycolique, l'acide thiolactique, la cystéine, les dérivés de la cystéine, la cystéamine et les dérivés de la cystéamine.

14. Composition selon l'une quelconque des revendications 11 à 13 **caractérisée en ce que** le ou les thiols représentent de 0,001 à 30 % en poids par rapport au poids total de la composition.

15. Composition selon la revendication 11 **caractérisée en ce que** le ou les disulfures représentent de 0,001 à 30 % en poids par rapport au poids total de la composition.

16. Composition selon la revendication 11 **caractérisée en ce que** le ou les sulfites ou bisulfites représentent de 0,001 à 30 % en poids par rapport au poids total de la composition.

17. Composition selon l'une quelconque des revendications précédentes **caractérisée en ce qu'**elle comprend au moins un chélateur de métaux.

18. Composition selon la revendication 17 **caractérisée en ce que** le ou les chélateurs de métaux sont choisis parmi l'acide éthylène diamine tétracétique (EDTA) et ses sels de sodium, de potassium, de calcium, de disodium, de diammonium et de triéthanolamine (TEA-EDTA), l'acide hydroxyéthyl éthylène diamine tétracétique (HEDTA) et son sel trisodique, et la néocuproine.

19. Composition selon la revendication 17 ou 18 **caractérisée en ce que** le ou les chélateurs de métaux représentent de 0,00001 à 10 % en poids par rapport au poids total de la composition.

20. Composition selon l'une quelconque des revendications précédentes **caractérisée en ce qu'**elle comprend au moins un agent réducteur autre que les thiols, dithiols, sulfites et bisulfites définis dans les revendications 11 à 16.

21. Composition selon la revendication 20 **caractérisée en ce que** le ou les agents réducteurs sont choisis parmi les (C₁-C₈) alkyl-phosphines et les réductones.

22. Composition selon la revendication 21 **caractérisée en ce que** les réductones sont choisies parmi l'acide ascorbique et l'acide érythorbique.

23. Composition selon l'une quelconque des revendications 20, 21 ou 22 **caractérisée en ce que** le ou les agents réducteurs représentent de 0,001 à 30 % en poids par rapport au poids total de la composition.

24. Composition selon l'une quelconque des revendications précédentes **caractérisée en ce qu'**elle comprend au moins un solvant choisi parmi les alcools en C₁-C₆, de préférence les alcanols tels que l'éthanol, le propanol et l'isopropanol, les alcanediols tels que l'éthylèneglycol, le propylène glycol et le pentanediol, l'alcool benzylique, les éthers en C₁-C₆, les esters en C₁-C₆, la N-méthylpyrrolidone (NMP), et les cétones en C₁-C₆.

25. Composition selon l'une quelconque des revendications précédentes **caractérisée en ce que** le pH de la composition est compris entre 2 et 13.

26. Composition selon l'une quelconque des revendications précédentes **caractérisée en ce qu'**elle comprend un ou plusieurs adjuvants cosmétiques choisis parmi les gérants et/ou épaississants, les tensioactifs anioniques, non ioniques, cationiques ou amphotères, les agents propénétrants, les émulsionnants, les parfums, les conservateurs, les charges, les filtres solaires, les matières colorantes, les protéines, les vitamines, les provitamines, les polymères non fixants anioniques, non ioniques, cationiques ou amphotères, les agents hydratants, les émollients, les agents adoucissants, les huiles minérales, végétales ou synthétiques, les actifs hydrophiles ou lipophiles comme les céramides et les pseudocéramides, les agents anti-mousse, les agents antiperspirants, les agents anti-radicaux libres, les polymères fixants ou non, les agents bactéricides, et les agents anti-pelliculaires.

27. Procédé de déformation permanente des fibres kératiniques, en particulier des cheveux, comprenant les étapes suivantes :
- appliquer sur les fibres kératiniques une composition telle que définie dans l'une quelconque des revendications 1 à 26 ;
- laisser poser ladite composition et ladite solution, à température ambiante ou à une température comprise entre 20 et 100°C ;
- rincer les fibres kératiniques ;
- fixer les fibres kératiniques, soit par mise en contact desdites fibres avec l'oxygène de l'air, soit par application d'une composition comprenant au moins un agent oxydant.

28. Procédé selon la revendication 27 **caractérisé en ce que**, après l'application de la composition telle que définis dans l'une quelconque des revendications 1 à 28 et avant de laisser poser, on applique une solution comprenant au moins un sel et/ou oxyde métallique, de préférence un sel et/ou oxyde de Cu(I), Cu(II) ou Fe(II).

29. Procédé selon la revendication 28 **caractérisé en ce que** le ou les sels et/ou oxydes de Cu(I), Cu(II) ou Fe(II) sont choisis parmi Cu₂NO₂, Cu₂O, CuSO₄, CuBr, FeO, FeCl₂ et Fe(C₃H₅O₃)₂.

30. Procédé selon la revendication 28 ou 29 **caractérisé en ce que**, après avoir laissé poser et avant les étapes de rinçage et de fixation, on applique une solution comprenant au moins un chélateur de métaux, et on laisse poser à température ambiante ou à une température comprise entre 20 et 100°C.

31. Kit cosmétique pour la déformation permanente des fibres kératiniques, **caractérisé en ce qu'**il comprend une première composition cosmétique telle que définie dans l'une quelconque des revendications 1 à 28 et une seconde composition cosmétique comprenant au moins un sel et/ou oxyde métallique, de préférence un sel et/ou oxyde de Cu(I), Cu(II) ou Fe(II), ladite première composition et ladite deuxième composition étant conditionnées séparément dans des compartiments imperméables à l'oxygène.

32. Kit selon la revendication 31 **caractérisé en ce qu'**il comprend une troisième composition comprenant au moins un chélateur de métaux, ladite troisième composition étant conditionnée séparément dans des compartiments imperméables à l'oxygène.
